# EUROPEAN PATENT APPLICATION

(11) **EP 3 035 317 A1**
(43) Date of publication of application: **22.06.2016**
(21) Application number: 14199492.1
(22) Date of filing: 19.12.2014
(51) Int. Cl.: G09B 5/06, A61B 5/00

(54) **Cognitive load balancing system and method**

(71) Applicant: Zoaring Adaptive Labs Limited, London N1 6AS (GB)
(72) Inventor: JORMELAND, Vegard, London, N1 5PH (GB)
(74) Representative: Miller Sturt Kenyon

(57) **Abstract**

An information presentation system comprising: a presentation apparatus for presenting predetermined information to a user; a measurement apparatus for continuously measuring physiological parameters of the user; a load determining apparatus for determining in real time an indication of a cognitive load experienced by a user based on the measured parameters; and an adapter configured, during presentation of the predetermined information, to successively adapt the presentation of the predetermined information based on the determined indication.

## Description

The present invention relates to a system and method for balancing the cognitive load experienced by the user. In preferred embodiments, the present invention is helpful in optimizing learning of a user.

In the current understanding of the human mind, it is generally considered that when a person learns information, the learnt information is stored in long term memory. The learnt information in semantic long-term memory is stored in the format of a schema of data. As a simple example, the schema could be for a tyre or a car. The schema for a car is likely to include the schema for a tyre as a sub- or linked schema. Schemas have no fixed size and they may be very large for an expert on a given subject or very small for a novice.

It is generally understood that in the course of learning, information is processed in a cognitive mechanism known as working memory and passed into long term memory.

One of the main functions of working memory may be to create and structure schemas into hierarchies, for example to make the tyre schema part of the larger car schema. However, the capacity of working memory is limited with the effect that there is a maximum number of schemas it can hold (but not how large the schemas are), and also a maximum of how much a person is able to think/process information per unit time. The amount of thinking/processing that takes place per unit time may be termed the cognitive load. The amount of the cognitive load is a result of how much capacity is being used in working memory.

The maximum cognitive load will vary for individual to individual. Moreover, the amount of cognitive load caused by a specific stimulus is dependent on the individual's prior experience and the capacity of their working memory. Thus, for some individuals a specific piece of information (say a theory from quantum physics) might induce a high cognitive load, whilst for others who are experts on quantum physics the same piece of information might induce a relatively low cognitive load. Furthermore, the cognitive load might fluctuate as the individual is exposed to different aspects of the theory, as a function of how well they know those specific aspects. Similarly, experienced drivers will experience a lower cognitive load than learner drivers, and the load will vary as the road conditions vary.

KR 2013-0130538 and US 2013/0325482 both disclose estimating cognitive load experienced by drivers of automobiles using electrocardiography and recognition of changes in the use of the spoken word respectively.

US 2014/0208226 discloses the assessment of the cognitive load of a reader of a digital document by tracking the reader's pupillary response to content located on a web page. The cognitive load may be aggregated for a user over time and for different portions of a displayed page. The information can used to determine how pages displayed in different sizes and by different web browsers are received and to show different users different adverts on the same page.

WO 2013/123587 discloses a perceptual cognitive motor learning system. The user's cognitive abilities are assessed as he does different physical tasks, based on input from the user, and the results are used to pace a physical training program.

According to a first aspect of the present invention, there is provided an information presentation system comprising: a presentation apparatus for presenting predetermined information to a user; a measurement apparatus for continuously measuring physiological parameters of the user; a load determining apparatus for determining in real time an indication of a cognitive load experienced by a user based on the measured parameters; and an adapter configured, during presentation of the predetermined information, to successively adapt the presentation of the predetermined information based on the determined indication.

Preferably, the physiological parameters are measured by at least one of EEG, ECG, eye tracking, and pupillary response.

More preferably, the load determining apparatus is configured to use a computation model having the measured parameters as inputs to determine the indication.

More preferably, the computational model comprises at least one of a neural network and a support vector machine.

More preferably, the system has a training mode for use before or after the presentation of the predetermined information for adapting the computational model to the user.

More preferably, the training mode is adapted to run a calibration routine, the calibration routine exposing the user to material arranged to systematically manipulate the cognitive load of the user.

More preferably, the training mode uses as inputs physiological parameters measured by the measuring means and rating information, which is provided by the user and is indicative of the cognitive load.

Preferably, the predetermined information comprises video; and the adapter is configured to change the playback speed of the video based on the indication.

Preferably, the predetermined information comprises audio; and the adapter is configured to change the playback speed of the audio based on the indication.

Preferably, the predetermined information comprises video and audio; and the adapter is configured to change the playback speed of at least one of the video and the audio based on the indication.

Preferably, the adaptation means is configured to adapt the presentation of the predetermined information by at least one of repeating presentation of at least part of the information and skipping presentation of at least part of the information.

According to another aspect of the present invention, there is provided a method for changing the cognitive load experienced by a user comprising: presenting predetermined information to a user; continuously measuring physiological parameters of the user; determining in real time an indication of a cognitive load experienced by a user based on the measured parameters; and during presentation of the predetermined information, successively adapting the presentation of the predetermined information based on the determined indication.

Embodiments of the present invention will now be described by way of example only with reference to the accompanying drawings, in which:
Fig. 1 is an illustration of variation in cognitive load experienced by a person;
Fig. 2 is a schematic drawing of a system according to the present invention;
Fig. 3 is a flowchart of a method of the present invention; and
Fig. 4 is a flowchart of another method of the present invention.

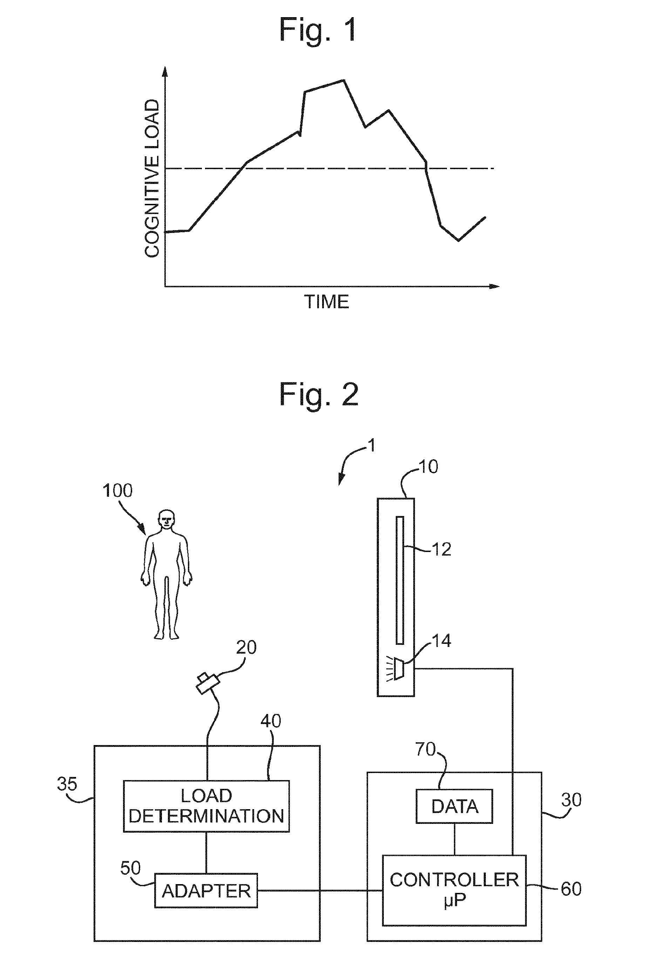

Fig. 1 shows a hypothetical example where an individual is exposed to information over time, such as an educational video. The solid line shows the required cognitive load for the individual to fully comprehend and process all the information presented in the video. However, the dotted line shows the individual's cognitive capacity - in other words, the individual's maximum cognitive load. From Fig. 1, where the required cognitive load is below the cognitive capacity (at the start and end of the video playback), the individual is not using his full processing capacity thus wasting processing resource and not learning as efficiently as possible.

By contrast, where the required cognitive load is above the cognitive capacity, the individual is being exposed to more information than he is able to process and comprehend. This is illustrated in the middle section of video playback, where the necessary processing (the required cognitive load) is greater than individual's cognitive capacity. The only way for the individual to fully comprehend the information in this case would be to repeat the exposure to the information. This is time consuming, taking double the amount of time to cover the information. Moreover, since the overall cognitive load required to comprehend and process the information a second time around would decrease in view of the individual's recent learning, there may be considerable wasted processing resource as the required load would be significantly below the cognitive capacity for extended periods. Put another way, because the particular user cannot process all of the exposed information sufficiently on first watching the video, the material is ineffective. When this problem is solved by repeating the video, a problem of inefficiency arises. It is therefore apparent that an individual's learning is often inefficient, leading to wasted time.

The present invention seeks to solve this problem by controlling the flow of information presented to the user in real time. In the example of Fig. 1 this would mean speeding up the flow of information (that is, speeding up the video) at the start and end of playback so that the required cognitive load approaches the working memory capacity, and slowing down playback in the middle section to bring the required cognitive load just below the working memory capacity. It is preferred that the cognitive load is kept as much as possible just below the working memory capacity, and as close as possible to it.

Accordingly, in a first embodiment the present invention provides a video playback system 1 as shown in Fig. 2. The system 1 comprises a presentation apparatus 10 comprising a display 12 for presenting video information and speakers 14 for presenting audio information. The presentation apparatus 10 may be an ordinary television, for example, as is well known in the art.

The presentation apparatus 10 is connected to a playback device 30, which includes a video and audio data store 70 and a controller 60, which controls and outputs data in a suitable format for presentation by the presentation apparatus 10. Thus, the playback device 30 may have similar functionality for example to any one or more of a CD player, DVD player, digital video recorder, internet or television radio service etc., all of which are well-known in the art. Thus, the playback device 30 will incorporate the required hardware and software components for outputting video and audio data to the presentation apparatus 10, including application, middleware and OS layers, data storage devices, video and audio codecs, multiplexers, engines, tuners etc., as known in the art, depending on the intended playback functionality.

As shown in Fig. 2, a user 100 views the video and listens to the audio output by the presentation apparatus 10. While the viewer is watching, predetermined physiological parameters of the user 100 are continually (in other words, at regular intervals) or continuously (in other words, without stopping) measured by a measuring apparatus 20. In this specification, the expressions 'continuous' and ' continual' may be considered as synonymous and incorporate both meanings attribute above. Like expressions should be construed accordingly. The measured parameters are not particularly limited, so long as they can be used to determine the cognitive load experienced by the user in real time, and will be discussed in more detail later.

The measured parameters are output from the measuring apparatus 20 to a cognitive load balancing apparatus 35, which includes a cognitive load determination component 40 and an adapter 50. The cognitive load determination component 40 receives the measured parameters and determines the cognitive load placed on the user by watching the video in real time. The result of the determination is output to the adapter 50.

Depending on a comparison between the determined cognitive load at a particular point in time and a predetermined threshold, the adapter sends a signal to the controller 60 of the playback device 60 to increase or decrease the playback speed. As the playback speed increases, so the cognitive load on the user 100 will increase. If it increases too much, the threshold will be reached and the adapter 50 will instruct the controller 60 to reduce the playback speed. Similarly, as the playback speed is decreased, so the cognitive load on the user 100 will decrease. If it decreases too much, the threshold will be reached and the adaptor 50 will instruct the controller 60 to increase the playback speed. In this manner, the system 1 operates as a feedback loop to maintain the cognitive load on the user 100 at around the threshold, which is preferably set just below the working memory capacity (or expected working memory capacity) of the user 100. In this manner, the processing demands associated with the information (the video and audio) match the working memory processing capacity of the user 100. This ensures that the user consistently uses his maximum processing resource without becoming overloaded, thereby increasing the efficiency of learning. However, the present invention is not limited to the use of a single threshold or to controlling the change in speed based on a single threshold in the manner described above. Alternatives will be explained below.

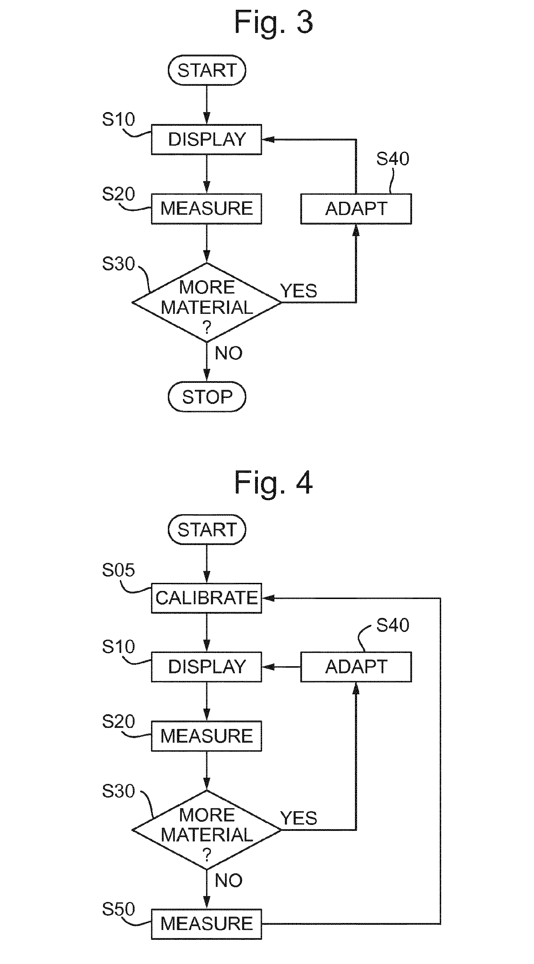

A related method of the present invention according to one embodiment is illustrated in Fig. 3, in which the presentation apparatus 10 is used for example to display video to the user 100 in step S10. In step S20, the user's physiological parameters are measured using the measuring apparatus 20. In S30, the balancing apparatus 35 determines from the playback device 30 whether there is further information to be presented. If there is, the balancing apparatus 35 controls the playback apparatus to change the presentation of information in step S40 based on the measured parameters.

As noted above, any one or more of a number of physiological parameters may be measured. The cognitive load experienced by a person affects physiological parameters in a fairly regular pattern. Thus, by measuring these patterns relevant psychological constructs, such as cognitive load, can be inferred. A range of measurement techniques may be used for this purpose, either individually or in combination. These include:
• Electroencephalography (EEG): electrodes placed on the scalp at systematic positions on the scalp can be used to indicate synaptic activity measurable at those locations. EEG markers unique to the individual may be detected during a calibration process (to be discussed later) and analysed using machine learning or pattern recognition techniques such as neural networks or support vector machines. The analysis may yield an indication of the synaptic activity corresponding to the cognitive load of the user, and how the synaptic activity varies as the cognitive load experienced varies. Both the quantity of synaptic activity and qualitative aspects such as characteristic patterns in the measured signals (known as EEG markers) may be used to predict cognitive load. The recorded synaptic activity typically falls within predetermined frequency bands, for example where alpha activity is from 7.5 -13 Hz and theta activity is from 4 - 7.5 Hz. As one possibility, cognitive load may be determined as a function of (alpha + theta) activity. Other variations also fall within the scope of the present invention and weighting may be given to the different locations on the scalp where different activity is measured.
• Electrocardiogram (ECG): both traditional invasive ECG and camera-based ECG may be used. Cognitive load may be estimated by analysis of, for example, heart rate variability. It has been found that, after pre-processing, video from a camera can be used a photoplethysmograph (PPG) to provide the cardiovascular pulse wave as is well-known in the art. The pre-processing may involve, for example, using a skin detection mask to collect only pixels that contain the PPG signal, and combining these with the 'u' component of the Luv colour space obtained by converting the RGB colour space of each frame. This yields a signal robust to motion, light and sampling variability. Both the instantaneous pulse rate (a moving average) and the fluctuation in amplitude (reflecting peripheral vasoconstriction variation) can be recovered from the PPG signal to determine an indication of change in cognitive load. For example, heart rate tends to increase gradually as cognitive load increases. Various indications of cognitive load can be extracted in a variety of ways from heart rate signals, as is well known in the art. One example is given in 'Remote detection of mental workload changes using cardiac parameters assessed with a low-cost webcam', Frédéric Bousefsaf et al, Computers in Biology and Medicine 53 (2014) 154-163. Any suitable method can be used however.
• Eye tracking: cognitive load may be estimated by analysis of eye movements, such as fixation times, as is known in the art.
• Pupillary response: cognitive load may be estimated by analysis of variation in pupil size.
• Dual task: cognitive load may be estimated by measuring the reaction time associated with shifting controlled attention to a secondary stimuli with random onset throughout the exposure to the materials.
• Other measures taken direct from the brain, including functional near infrared imaging, transcranial Doppler sonography, magnetoencephalography, and functional magnetic resonance imaging.
• Other indirect measures including skin conductance, galvanic skin response and other electrodermal activity.

In the present invention, the preferred method of capturing physiological data is camera-based ECG. Thus, in preferred embodiments, the measuring apparatus 20 is a camera and the load determination apparatus 40 pre-processes the video and extracts PPG data to determine the moving average pulse rate and fluctuations in amplitude to determine the cognitive load of the user 100.

The data collected by the measurement apparatus can be analysed in the cognitive load determination apparatus using pattern classification or machine learning algorithms, in which the measured data is compared with data that has been classified previously. The skilled addressee will recognise that any suitable algorithm may be used, however. As data is accumulated, changes in the measured data can be used to determine changes in the cognitive load experienced by the user. Either these changes alone can be used to adapt the presentation of the data (speed up or slow down playback), or the determined cognitive load can be compared to a threshold as described above. The threshold may be established based on an initial training/calibration phase discussed below, or it may be set at a previously fixed level. In either case, the calibrated or initially fixed threshold may be varied as the presentation of information progresses (as the video plays) or as post-presentation feedback/measurement is input into the system.

The system 1 may use one or a group of algorithms that automatically develop models from the data. This is known as machine learning and is a field of artificial intelligence. Machine learning nominally enables a program to learn from data, objectively improving performance without explicit human interference or programming beyond the initial algorithm and inputs.

In addition, or instead, the system 1 may use pattern recognition. This includes at least one of deterministic pattern matching and probabilistic pattern matching. Rather than simply classifying input discretely, pattern recognition algorithms also output real number predictions, and hence are virtually equivalent to matching learning except in the explicit expectation of iterative learning through additional empirical data. Increasingly, pattern recognition can be considered as being included in or subsumed in machine learning.

In any event, the purpose is to apply computational techniques to develop accurate models of a given system on the basis of empirical data. The model predicts the future behaviour of the system with a quantifiable accuracy. An initial model can be generated from initial (training) data and further test data can be used to test and increase the predictive accuracy of the model. This is typically done by minimising a cost function, which penalises a given model's failures to correctly predict a measured output variable on the basis of known input variables.

One example of an analysis technique suitable for use in the present invention is the use of neural networks. In this approach, a classifying decision rule is conceptualised as a network of idealised neurons. Each neuron has a number of inputs (analogous to dendrites) and one output (analogous to an axon). The network is envisaged as a series of layers of parallel neurons, where each neuron in the outer layer receives all variables of each data point through the various input channels, and combines the inputs into a single output which becomes an input for the next layer. Each neuron component acts lie the logistic regression, a sigmoid function of the input variables.

The quantitative operation of each neural network layer can be represented by a matrix, and thus the neural network model can be computed as a sequence of matrices operating on the data point vector.

The common didactic example of neural networks is a funnel with a single neuron in the innermost layer that outputs a single variable, a normalised probability between 0 and 1, which is used to classify data with a discrete two level label. However, neural networks can in principle be used to predict any number of distinct variables. Nevertheless, neural networks may lend themselves best to predicting variable with finite ranges. The advantage of neural networks with respect to a simpler classifier such as logistic regression is that they will automatically emphasise potentially important features containing non-linear terms.

Another example of an analysis technique suitable for use in the present invention is the use of vector machines. These often offer a computationally simpler and predictively more effective approach than neural networks. Support vector machine (SVM) algorithms may be heuristically thought of as large margin classifiers. SVMs do not output probabilistic predictions, but rather deterministic discrete classification. The term large margin classifier is used because partly the motivation behind SVMS is not only to classify different parts of the data set, but also to minimise the over fitting (bias) due to training data idiosyncrasies. While the SVM classifier produces a decision boundary in the same way as logistic regression and neural networks-which is generalised to a hyper plane for higher dimensional spaces- it also sets this decision boundary with a view towards maximising the margin between the boundary and the training data pints on each side of the boundary.

Both neural networks and SVMs automatically choose relevant features, or basis functions, such as linear combinations of non-linear terms involving the input variables. However, SVMs optimise over a provably convex cost function, eliminating the possibility of local minima and focusing always on a global solution for the given data set.

SVMs are considered to be a sparse kernel method, which means that the small subset of the original training data vectors is used directly in the construction of the predictive machinery rather than only aggregates of the training data.

In the present invention, the use of SVMs is preferred to process the physiological data recovered from the camera 20 to determine an indication of the cognitive load experienced by the user 100.

In summary, any suitable physiological data may be measured and used to determine the cognitive load experienced by a user 100, although non-invasive ECG data is preferred. The determination itself may be made by any suitable technique, although machine learning techniques such as neural networks or support vector machines are preferred, with the use of SVMs being particularly preferred. In such techniques a model is created and trained using initial data. The determination is compared to a predetermined threshold, which itself may be established by training the model, and the result of the comparison is used to change the presentation of information.

In this specification, the concept determining the cognitive load and comparing the result to a threshold should be interpreted broadly and encompasses establishing whether a particular set of physiological values, including a particular change or set of changes in these values, indicates that the user's necessary processing is below or above a predetermined working memory capacity based on previously input data. Alternatively, the concept includes determining a change in cognitive load and changing the presentation of information (the speed of video playback) as a result of the change in cognitive load.

In initial training of the system, a user may be exposed to material arranged to systematically manipulate the cognitive load of the user-for example, the user may be exposed to information that is initially very easy to understand but becomes progressively harder to understand. The user provides feedback as to how easy the information was to understand at fixed points and this is compared to physiological measurements taken from the user. With increased data points, and with increased numbers of users being sampled, by comparing physiological measures of cognitive load with subjective rating scales (such as NASA TLX), it is possible to determine different levels of cognitive load with relatively high validity during the initial training phase.

In the foregoing description, at least in initial use of the system, the predetermined markers of cognitive load are not specifically matched to the specific user 100 and the way cognitive load is determined is not tailored to variations in the way the individual experiences changes in cognitive load physiologically. To avoid this, all training of the model of a particular system 1 can be carried out using only the user 100 or a system initially trained with a plurality of users can be fine tuned by training with only the user 100. In particular, before use the system may be calibrated to the user 100. This is achieved by exposing the user to material arranged to systematically manipulate his cognitive load, while measuring the predetermined physiological parameters, and obtaining feedback from the user on his cognitive load at fixed points during the exposure (in the same way that the system model is initially trained). The results may be used to calibrate the system 1 to the specific user.

Alternatively, in a preferred embodiment of the present invention, illustrated with reference to Fig. 4, the system 1 and method further incorporate at least one of an initial calibration/training step S05 and a post-use updating step (shown as a measuring step S50 combined with a calibration step S05 in Fig. 4).

Similarly, after the information has been presented (in other words, the video has been played), the user may again be exposed to the training information and the physiological and user inputs may be used to further refine the model and adapt it to the specific user. By implementing the pre- or post-calibration processes, the system 1 can better learn the individual user's markers of cognitive load and when his cognitive load approaches or reaches working memory capacity-that is, when demands for processing (thus necessary cognitive load to achieve a certain processing outcome) per time unit approach or exceed the working memory capacity. In this manner the presentation of information (video playback) becomes better attuned to the individual user 100. This may involve for example exposing the user to the NASA-TLX or other subjective rating scale of cognitive load, and using the classification of cognitive load from the NASA-TLX to train the system in combination with the physiological data collected during exposure to the materials.

As previously explained, in embodiments of the present invention, the speed of video playback is increased if the determined cognitive load is sufficiently below the user's working memory capacity, and is decreased if it is below but sufficiently close to, or above, the user's working memory capacity. In one embodiment, the speed is set at a single higher speed of, for example 1.5 x normal speed if the determined cognitive load is too low, at normal speed if cognitive load is in an optimum range or level, and at a single lower speed of, for example, 0.75 x normal speed if cognitive load is too high. However, the speed may be varied on a continuum as cognitive load varies away from the optimum range or level, with increased distance from the optimum resulting in greater variation from the normal playback speed. Similarly, the speed may be varied in a series of increments as cognitive load varies away from the optimum range or level, with increased distance from the optimum again resulting in greater variation from the normal playback speed. This can be achieved by having a number of thresholds (or ranges of determined cognitive load) and setting a predetermined speed for each threshold, or a predetermined change in speed (eg increase speed by 10% or lower speed by 15%) for each threshold.

In a preferred embodiment the machine learning algorithm/pattern classifying algorithm is used to classify the physiological signal with regards to the amount of the cognitive load. The algorithm then outputs one number which can be called a cognitive load index. This cognitive load index could be made to fall within a predetermined range of for example hundreds of numbers, in which each number would be associated with a specific and distinct level of cognitive load.

This determined cognitive load index could then be compared to a threshold. The threshold may be a single number at which the balance of cognitive load to working memory capacity is considered to be optimum. Alternatively, the threshold may be two or more numbers (assuming that the threshold state is not homogenous) or a range of numbers, or it could be no numbers (thus right between two numbers, which are classified as underload and overload respectively). Based on the cognitive load index and the threshold, it is then possible to make an accurate prediction of the exact amount of increase/decrease of speed and consequently cognitive load to arrive at or approach the threshold. If the cognitive load index equals the threshold or falls within the threshold range, the cognitive load index is considered optimum and the speed is not changed.

The amount of change of speed may be fixed irrespective of distance of the cognitive load index from the working memory capacity. In other words, whatever the distance of the cognitive load index from the working memory capacity, the speed will increase or decrease a fixed amount. Preferably, however, the amount in the change in speed of playback can be made to depend on the specific cognitive load index that is being determined at any one instant. Generally, the closer the cognitive load index is to the working memory capacity threshold, the smaller the change in speed. The amount of change of speed may vary (accelerate) linearly with increasing distance of the cognitive load index from the cognitive capacity; or it may vary (accelerate) non-linearly with increasing distance of the cognitive load index from the cognitive capacity. Varying the amount of the change in speed allows adaptions to the speed of playback to be done more quickly, and only one adaption per fluctuation in cognitive load may be needed to arrive at or approach the threshold, as opposed to several if the physiological signal is simply classified as either underload or overload (without more fine grained classes within them).

In each case, it is preferred that the maximum variation in playback speed be capped - if playback speed is too fast or too slow, it becomes effectively or actually unintelligible, irrespective of the cognitive load.

By measuring cognitive load in real time using psychophysiological measures, and then adjusting the cognitive load by changing the speed of playback, the user's working memory can be more optimally used. This has two benefits. First, in conditions where there would have been underload (wasting of processing resource) the video will be speeded up. This allows the user to achieve the same quantity and quality of learning in less time. Second, in conditions where there would have been overload, the video will have been slowed down. This increases the amount of time and hence processing resource available to process the learning materials. Thus, the individual will have enough time and consequently resource to attend to and learn all learning materials, not leaving holes in his knowledge. This assures quality of learning.

In the foregoing, it has only been described that the playback speed of the video is varied with the change in cognitive load. In preferred embodiments, the audio playback speed is changed in the same manner. However, it is not necessary that video and audio playback speed be varied by the same amount. If one is varied more than the other, the faster playing medium can be paused at a suitable juncture until the other has caught up. In the meantime, additional material (audio or visual) can be presented to occupy the user. In a similar manner, it is possible to adjust the playback speed of the video or audio only.

In addition, where cognitive load is determined to be particularly high, complicated information may be skipped - that is, not presented - as being too difficult for the user to understand. In addition or instead, if processing demands are above the working memory capacity and the material is being displayed at the lowest speed, it can be inferred that the participant does not have enough prior knowledge to comprehend the material. The participant will therefore be exposed to simpler related materials, which in turn will make him able to comprehend the more advanced material when he is subsequently re-exposed to it.

Similarly, where cognitive load is determined to be particularly low, information may be skipped as being too simple and implicitly already known by the user. In addition, or instead, where cognitive load is consistently low, additional information may be presented, which is most likely more complicated and likely to better engage the user.

The present invention has been described thus far with reference to video and audio information. However, it also encompasses the presentation of information in other ways, including the presentation of pictures, slideshows, presentation slides/images (e.g. PowerPoint presentations), text and information in any other format.

The foregoing description concentrates on changing the speed of video or other presented information in real time. However, the present information can also be used to adapt what material is presented in real time, for example by skipping or adding to the amount of information presented to the user based on his cognitive load.

Although in the foregoing description the playback device 30 and the balancer 35 are shown as separate items, they may be integrated into a single unit. The controller 60 may carry out one or more of the functions of load determination and control of adapting the presentation of the information. Either or both the playback device and the balancer 35 may be implemented using a standard computer, such as a laptop/tablet. In addition, the measurement apparatus (camera) 20 may be built in to the computer or provided separately. In the same way, the display 12 and the speakers 14 may built in to the computer or provided separately. Alternatively, any or all of the components may be provided separately and any and all processing operations may be carried out be a distributed processor.

The foregoing description has been given by way of example only and it will be appreciated by a person skilled in the art that modifications can be made without departing from the scope of the present invention.

In this specification, the expression 'real time' is intended to indicate that the system receives constantly changing data, which is used to determine a constantly changing indication of cognitive load, and to adapt the presentation of information accordingly. However, there is no particular limitation in respect of required sampling and processing rates.

## Claims

1. An information presentation system comprising:
a presentation apparatus for presenting predetermined information to a user;
a measurement apparatus for continuously measuring physiological parameters of the user;
a load determining apparatus for determining in real time an indication of a cognitive load experienced by a user based on the measured parameters; and
an adapter configured, during presentation of the predetermined information, to successively adapt the presentation of the predetermined information based on the determined indication.

2. A system according to claim 1, wherein the physiological parameters are measured by at least one of EEG, ECG, eye tracking, and pupillary response.

3. A system according to claim 2, wherein the load determining apparatus is configured to use a computation model having the measured parameters as inputs to determine the indication.

4. A system according to claim 3, wherein the computational model comprises at least one of a neural network and a support vector machine.

5. A system according to claim 3 or claim 4, having a training mode for use before or after the presentation of the predetermined information for adapting the computational model to the user.

6. A system according to claim 5, wherein the training mode is adapted to run a calibration routine, the calibration routine exposing the user to material arranged to systematically manipulate the cognitive load of the user.

7. A system according to claim 5 or claim 6, wherein the training mode uses as inputs physiological parameters measured by the measuring means and rating information, which is provided by the user and is indicative of the cognitive load.

8. A system according to any one of the preceding claims, wherein:
the predetermined information comprises video; and
the adapter is configured to change the playback speed of the video based on the indication.

9. A system according to any one of the preceding claims, wherein:
the predetermined information comprises audio; and
the adapter is configured to change the playback speed of the audio based on the indication.

10. A system according to any one of the preceding claims, wherein:
the predetermined information comprises video and audio; and
the adapter is configured to change the playback speed of at least one of the video and the audio based on the indication.

11. A system according to any one of the preceding claims, wherein the adaptation means is configured to adapt the presentation of the predetermined information by at least one of repeating presentation of at least part of the information and skipping presentation of at least part of the information.

12. A method for changing the cognitive load experienced by a user comprising:
presenting predetermined information to a user;
continuously measuring physiological parameters of the user;
determining in real time an indication of a cognitive load experienced by a user based on the measured parameters; and
during presentation of the predetermined information, successively adapting the presentation of the predetermined information based on the determined indication.
